# EUROPEAN PATENT SPECIFICATION

(11) **EP 1 409 485 B1**
(45) Date of publication and mention of the grant of the patent: **26.04.2006**
(21) Application number: 02746260.5
(22) Date of filing: 02.07.2002
(51) Int. Cl.: C07D 471/04, C07D 491/052, C07D 495/04, A61K 31/4745, A61K 31/4162, A61P 3/10, A61P 11/06, A61P 17/06, A61P 19/02, A61P 37/02

(54) **IMMUNOMODULATING COMPOUNDS**
IMMUNMODULIERENDE VERBINDUNGEN
COMPOSES IMMUNOMODULATEURS

(30) Priority: 04.07.2001 SE 0102404
(43) Date of publication of application: 21.04.2004
(73) Proprietor: Active Biotech AB, 220 07 Lund (SE)
(72) Inventor: BJÖRK, Per, Axel, 256 56 Helsingborg (SE); FEX, Tomas, 222 52 Lund (SE); PETTERSSON, Lars, Olof, Göran, 247 31 Södra Sandby (SE); SORENSEN, Poul, 1054 Köpenhamn K (DK); DA GRACA THRIGE, Dorthe, 2500 Valby (DK)
(74) Representative: Henriksson, Dan Ragnar Mikael
(86) International application number: PCT/SE2002/001309
(87) International publication number: WO 2003/004495

(56) References cited:
- EP-A1- 0 354 693
- EP-A1- 0 354 694
- WO-A1-91/06298
- WO-A1-91/11448
- WO-A1-97/34893
- US-A- 4 268 516
- US-A- 4 312 870
- SAVINI L. ET AL.: 'High affinity central benzodiazepine receptor ligands: Synthesis and structure-activity relationship studies of a new series of pyrazolo(4,3-c)quinolin-3-ones' BIOORGANIC & MEDICINAL CHEMISTRY vol. 6, 1998, pages 389 - 399, XP002958413

## Description

### Field of the invention

The present invention relates to immunomodulating compounds, pharmaceutical compositions comprising said compounds, use of said compound as well as a method for treatment of medical conditions which benefit from immunomodulation, wherein said compounds are administered.

### Background of the invention

The immune system possesses the ability to control the homeostasis between the activation and inactivation of lymphocytes through various regulatory mechanisms during and after an immune response. Among these are mechanisms that specifically inhibit and/or turn off an immune response. Thus, when an antigen is presented by MHC molecules to the T-cell receptor, the T-cells become properly activated only in the presence of additional co-stimulatory signals. In the absence of accessory signals there is no lymphocyte activation and either a state of functional inactivation termed anergy or tolerance is induced, or the T-cell is specifically deleted by apoptosis.

One such co-stimulatory signal involves interaction of CD80 on specialised antigen-presenting cells with CD28 on T-cells, which has been demonstrated to be essential for full T-cell activation. (Lenschow et al. (1996) Annu. Rev. Immunol., 14, 233-258).

### Prior art

In US 4,312,870 compound A is disclosed as one of several psychoactive compounds but without any biological data. Some related compounds are described by A. Carotti in Bioorganic & Medicinal Chemistry 6 (1998) 389 - 399, and from data related to these compounds it is obvious that the carboxylic acid substituent greatly diminishes biologic activity measured as affinity for the CNS benzodiazepine receptor.

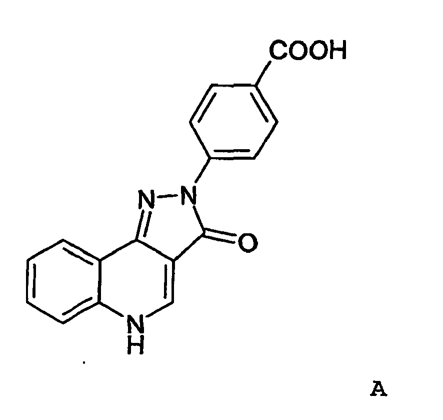

EP 0354693A1 (Boots) discloses immunomodulatory compounds of general structure B but does not include structures wherein R7 and/or R8 are COOH or contain a COOH group.

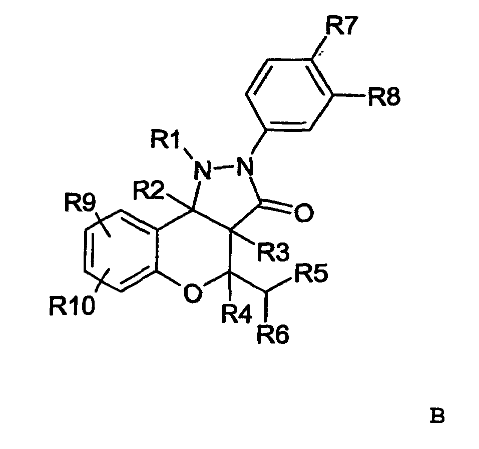

Similarly EP 0354694A1 (Boots) discloses immunomodulatory compounds of general structure C but no structures wherein R6 and/or R7 are COOH or contain a COOH group are described.

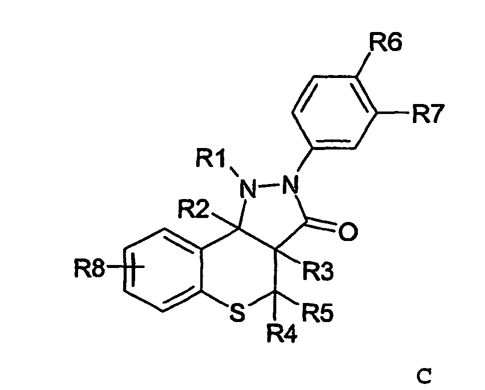

Also, WO9111448 (Boots) discloses immunomodulatory compounds of general structure D but here are no structures wherein R7 and/or R8 and R8' are COOH or contain a COOH group.

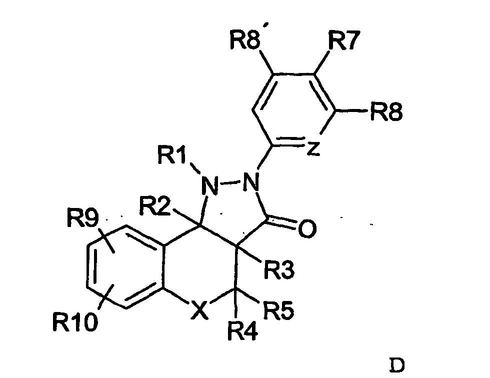

### Summary of the invention

The present invention relates in a first aspect to a novel compound having the general formula (I) 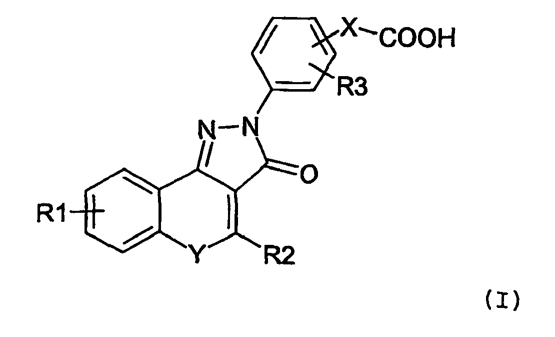 wherein X represents a bond or a group selected from substituted or unsubstituted C₁₋₃-alkyl, NH-C(O)-C₁₋₃₋alkyl, NH-C(O)-CH₂-O-CH₂ or C(O)-NH-(amino acid residue); Y represents NR4, O or S;
R1 represents H, halo, CF₃, lower alkyl or lower alkoxy; R2 and R4 represents independently H or lower alkyl; and
R3 represents H, halo, lower alkyl or lower alkoxy,
wherein halo is F, Cl or Br;
wherein lower alkyl represents saturated or unsaturated, straight, branched or cyclic alkyl groups having 1-6 carbon atoms; and
wherein lower alkoxy represents saturated or unsaturated, straight, branched or cyclic alkoxy groups having 1-6 carbon atoms,
with the proviso that R2 is not H, when X is a bond and Y is NH and R3 is H,
or pharmaceutically acceptable salts thereof.

In one preferred embodiment of the invention the compound X is a bond and in another preferred embodiment Y is NH.

In further embodiments the compound is selected from the group comprising
{[3-(3-Oxo-3,5-dihydro-pyrazolo[4,3-*c*]quinolin-2-yl)-phenylcarbamoyl]-methoxy}-acetic acid,
*N*-[4-(3-Oxo-3,5-dihydro-pyrazolo[4,3-*c*]quinolin-2-yl)-phenyl]-succinamic acid,
4-[4-(3-Oxo-3,5-dihydro-pyrazolo[4,3-*c*]quinolin-2-yl)-phenylcarbamoyl]-butyric acid,
{[4-(3-Oxo-3,5-dihydro-pyrazolo[4,3-*c*]quinolin-2-yl)-phenylcarbamoyl]-methoxy}-acetic acid,
4-[4-(3-Oxo-3,5-dihydro-pyrazolo[4,3-*c*]quinolin-2-yl)-phenylcarbamoyl]-2-phenyl-butyric acid,
*N*-[3-(3-Oxo-3,5-dihydro-pyrazolo[4,3-*c*]quinolin-2-yl)-phenyl]-succinamic acid,
2-{[4-(3-Oxo-3,5-dihydro-pyrazolo[4,3-*c*]quinolin-2-yl)-phenylcarbamoyl]-methyl}-benzoic acid,
2-Chloro-4-(3-oxo-3,5-dihydro-pyrazolo[4,3-*c*]quinolin-2-yl)-benzoic acid,
4-(6,8-Dimethyl-3-oxo-3,5-dihydro-pyrazolo[4,3-*c*]quinolin-2-yl)-benzoic acid,
4-(8-Methoxy-6-methyl-3-oxo-3,5-dihydro-pyrazolo[4,3-*c*]quinolin-2-yl)-benzoic acid,
4-(6,8-Dimethoxy-3-oxo-3,5-dihydro-pyrazolo[4,3-*c*]quinolin-2-yl)-benzoic acid,
4-(7,9-Dimethoxy-3-oxo-3,5-dihydro-pyrazolo[4,3-*c*]quinolin-2-yl)-benzoic acid,
4-(6-Methyl-3-oxo-8-trifluoromethyl-3,5-dihydro-pyrazolo[4,3-*c*]quinolin-2-yl)-benzoic acid,
4-(7,9-Dichloro-3-oxo-3,5-dihydro-pyrazolo[4,3-*c*]quinolin-2-yl)-benzoic acid,
[4-(3-Oxo-3,5-dihydro-pyrazolo[4,3-*c*]quinolin-2-yl)-phenyl]-propionic acid,
[4-(3-Oxo-3,5-dihydro-pyrazolo[4,3-*c*]quinolin-2-yl)-phenyl]-acetic acid,
4-(4-Methyl-3-oxo-3*H*-chromeno[4,3-*c*]pyrazol-2-yl)-benzoic acid,
4-(3-Oxo-3*H*-thiochromeno[4,3-*c*]pyrazol-2-yl)benzoic acid,
4-(5-Methyl-3-oxo-3,5-dihydro-pyrazolo[4,3-c]quinolin-2-yl)-benzoic acid,
2-[4-(3-Oxo-3,5-dihydxo-pyrazolo[4,3-c]quinolin-2-yl)-benzoylamino]-3-phenyl-propionic acid, and
2-[4-(3-Oxo-3,5-dihydro-pyrazolo[4,3-c]quinolin-2-yl)-benzoylamino]-2-acetic acid.

In yet another embodiment said compound is a CD80 antagonist, capable of inhibiting the interaction between CD80 and CD28.

The present invention relates in a second aspect to a compound as set forth above for use as a medicament.

In one preferred embodiment said compound is used as a medicament for treatment of medical conditions chosen from the group comprising rheumatoid arthritis, multiple sclerosis, diabetes, asthma, transplantation, systemic lupus erythematosis and psoriasis.

The present invention relates in a fourth aspect to a pharmaceutical composition comprising said compound as active ingredient in association with a pharmaceutically acceptable adjuvant, diluent or carrier.

In one prefered embodiment said pharmaceutical composition is used for treatment of medical conditions chosen from the group comprising rheumatoid arthritis, multiple sclerosis, diabetes, asthma, transplantation, systemic lupus erythematosis and psoriasis.

The present invention relates in a fifth aspect to the use of a compound having the general formula (I) 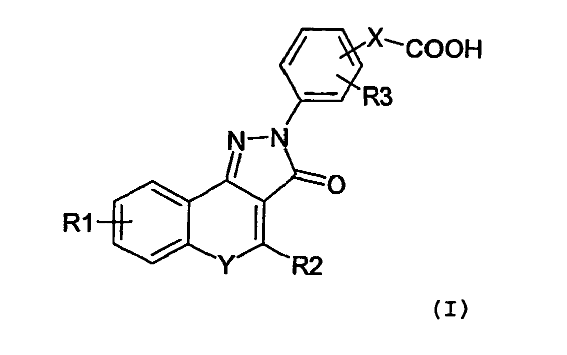 wherein X represents a bond or a group selected from substituted or unsubstituted C₁₋₃-alkyl, NH-C(O)-C₁₋₃₋alkyl, NH-C(O)-CH₂-O-CH₂ or C(O)-NH-(amino acid residue); Y represents NR4, O or S;
R1 represents H, halo, CF₃, lower alkyl or lower alkoxy; R2 and R4 represents independently H or lower alkyl; and
R3 represents H, halo, lower alkyl or lower alkoxy,
wherein halo is F, Cl or Br;
wherein lower alkyl represents saturated or unsaturated, straight, branched or cyclic alkyl groups having 1-6 carbon atoms; and
wherein lower alkoxy represents saturated or unsaturated, straight, branched or cyclic alkoxy groups having 1-6 carbon atoms, or pharmaceutically acceptable salts thereof, for the manufacture of a medicament for treatment of medical conditions which benefit from immunomodulation.

In one embodiment said medical conditions are chosen from the group comprising rheumatoid arthritis, multiple sclerosis, diabetes, asthma, transplantation, systemic lupus erythematosis and psoriasis.

In one embodiment said medical conditions are chosen from the group comprising rheumatoid arthritis, multiple sclerosis, diabetes, asthma, transplantation, systemic lupus erythematosis and psoriasis.

In another embodiment said therapeutically effective amount per day is within the range of 0,001-10 mg/kg body weight, preferably within the range of 0,1-5 mg/kg body weight.

### Detailed description of the invention

According to the present invention it has been found that compounds of general formula I are CD80 antagonists. Compounds of the general formula I inhibit the interaction between CD80 and CD28. The CD80 antagonistic properties of the compounds of general formula I have been established in Surface Plasmon Resonance (BIAcore) experiments.

It is preferred that X in the general formula I is a bond and in such cases other substituents, such as an adjacent meta-chloro, improves activity.

The compounds of the present invention may be in the acid form but may also be in the form of pharmaceutically acceptable salts.

The compounds of the present invention may also be in the form of prodrugs, especially esters with appropriate alcohols. Prodrugs can have improved pharmacokinetic and/or solubility properties.

Since the compounds of formula I are CD80 antagonists capable of interfering with the CD80 - CD28 interaction they are useful for treatment of inflammatory conditions and autoimmune diseases, e.g. rheumatoid arthritis, multiple sclerosis, diabetes, asthma, transplantation, systemic lupus erythematosis and psoriasis.

Effective quantities of the compounds of formula I are preferably administered to a patient in need of such treatment according to usual routes of administration and formulated in usual pharmaceutical compositions comprising an effective amount of the active ingredient and a suitable pharmaceutically acceptable carrier. Such compositions may take a variety of forms, e.g. solutions, suspensions, emulsions, tablets, capsules and powders prepared for oral administration, sterile solutions for parenteral administration, suppositories for rectal administration or suitable topical formulations. Conventional procedures for the selection and preparation of suitable pharmaceutical formulations are described, for example, in "Pharmaceuticals - The Science of Dosage Form Design", M. B. Aulton, Churchill Livingstone, 1988.

A suitable daily dose for use in the treatment of any of the conditions mentioned above is within the range of 0.001 mg/kg to 10 mg/kg body weight, in particular within the range of 0.01 mg/kg to 5 mg/kg body weight, depending upon the specific condition to be treated, the age and weight of the specific patient, and the specific patient's response to the medication. The exact individual dosage, as well as the daily dosage, will be determined according to standard medical principles under the direction of a physician.

Various additives to enhance the stability or ease the administration of the drug can be added to the formulation. The pharmaceutical composition may also contain additional therapeutically useful substances other than one or more compounds of the general formula I.

The present invention is further illustrated by the following non-limiting experimental part.

### Experimental part

The compounds of general formula I may be prepared by the methods described below. The prior art patent documents cited above also include useful synthetic methods.

Thus, compounds of general formula I wherein Y = N and X is an alkyl chain can be prepared as shown in the reaction below;

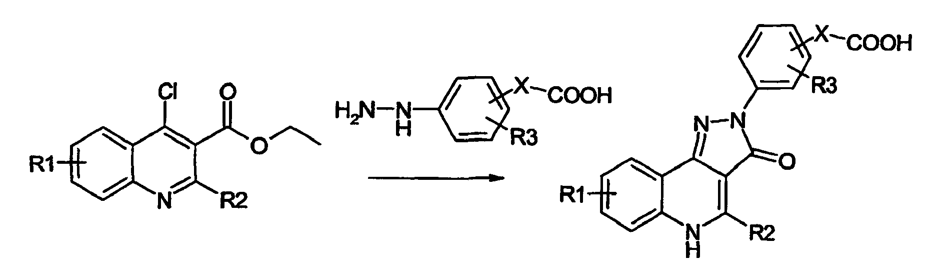

The starting material is available by known procedures (e.g. L. Savini et al, Bioorganic & Medicinal Chemistry 6 (1998) 389-399) and the reaction with hydrazine derivatives is performed by heating in a suitable solvent such as n-butanol. In cases where the acid is esterified under the reaction conditions, hydrolysis gives back the acid. Preparation of hydrazine derivatives was accomplished following literature procedures (Hunsberger et al, J. Org. Chem. 21 (1956) 394, 395, 396. Harden F. A. et al, J. Med. Chem. 34 (1991) 2892-8).

Compounds wherein R4 is lower alkyl may be obtained by subsequent alkylation. If the alkylation results in ester formation, the corresponding acid is easily obtained by hydrolysis.

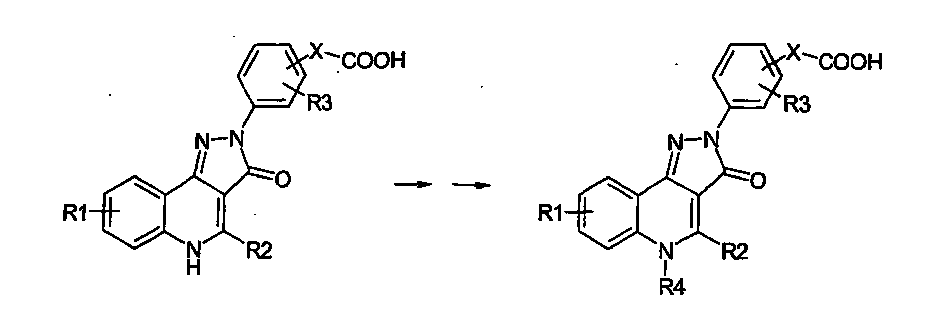

The compounds of general formula I wherein Y = O and X is an alkyl chain can be prepared according to various literature procedures (e.g. Ghosh C. K. Et al, Synthesis (1978) 779-781; Frogett J. A. et al, J. Chem. Research (S) (1997) 30-31). One synthetic route, when R2 = Me, is described below. The cyano group is subsequently hydrolysed to produce the corresponding acid.

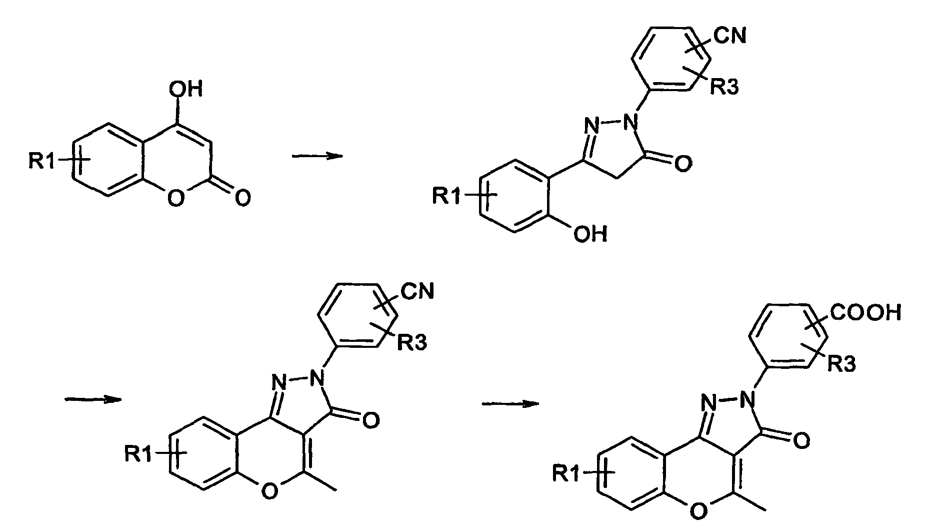

The compounds of general formula I wherein Y = S and X is an alkyl chain can be prepared as shown below using procedures described in the literature (Donelly M. X. D. et al, J. Chem. Soc. Perkin Trans. 1 (1993) 1729-1735; Lombardino J. G. et al, J. Med. Chem. 24 (1981) 830-834). The final oxidation was accomplished by stirring in air.

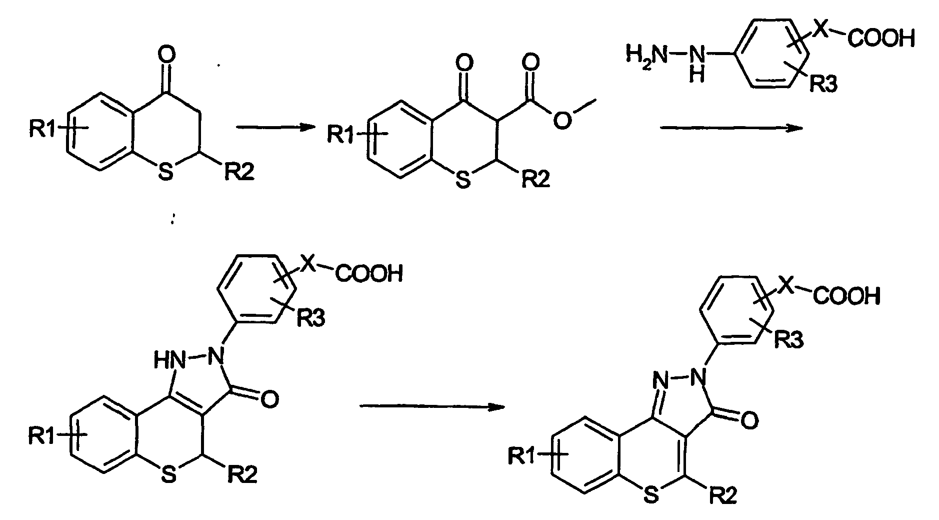

The compounds wherein X is a bond may also be obtained from the corresponding cyanide derivatives and the amino acid derivatives are prepared by condensation of the acid with the appropriate amino acid.

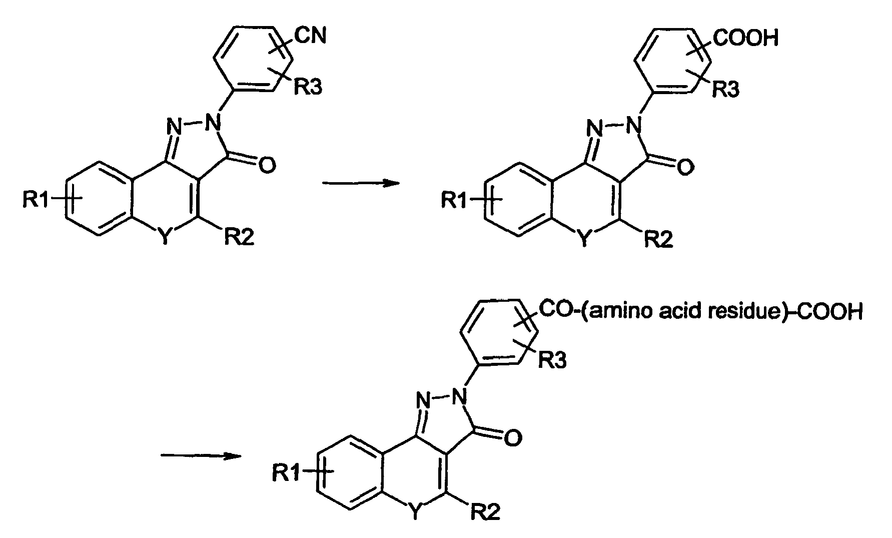

Compounds wherein X is NH-C(O)-(alkyl)- and NH-C(O)-CH₂-O-CH₂- can be prepared by reduction of the nitro group (L. Savini et al, Bioorganic & Medicinal Chemistry 6 (1998) 389-399) and subsequent acylation with the appropriate anhydride.

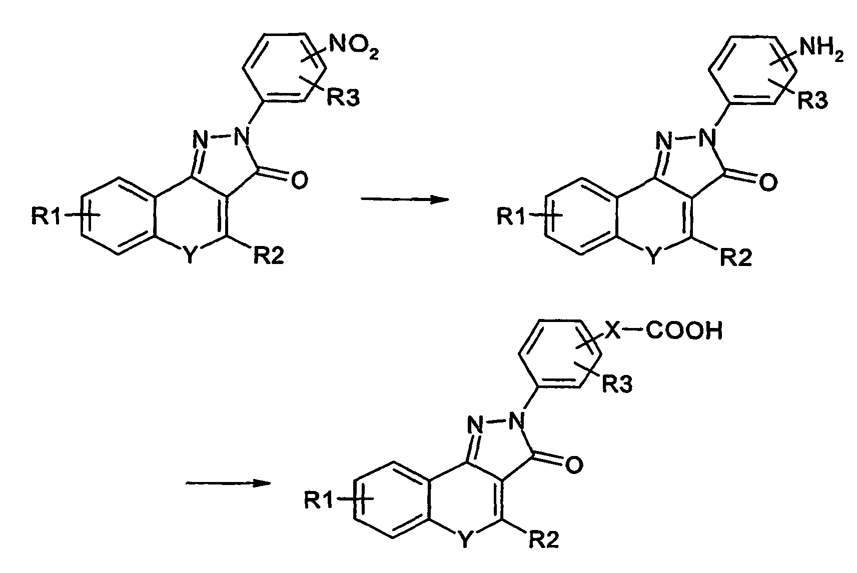

### Examples

The following examples are intended to illustrate the invention without restricting the scope thereof. Compounds were named using Autonom 2.1 from Beilstein. NMR spectra were recorded on a Bruker ARX 400 instrument. Coupling constants in the aromatic area are mostly referred to as singlets (s), doublets (d), triplets (t) in order to reflect the appearance of the NMR spectrum.

### Example 1

### {[3-(3-Oxo-3,5-dihydro-pyrazolo[4,3-c]quinolin-2-yl)-phenylcarbamoyl]-methoxy}-acetic acid

To a suspension of 2-(3-amino-phenyl)-2,5-dihydropyrazolo[4,3-*c*]quinolin-3-one (0.050 g, 0.18 mmol) in DMF (0.8 ml) diglycolic anhydride (0.025 g, 0.22 mmol) and 4-dimethylaminopyridine (0.007 g, 0.05 mmol) were added. The clear solution was stirred at room temperature and the product precipitated during the reaction. After 3 h water was added to the mixture. The precipitate was collected, washed with water and dried to yield {[3-(3-oxo-3, 5-dihydro-pyrazolo [4,3-*c*]quinolin-2-yl)-phenylcarbamoyl]-methoxy}-acetic acid (0.064 g) : ¹H NMR (400 MHz, DMSO-*d*_{*6*}) δ 12.8 (1H, bs), 10.02 (1H, s), 8.69 (1H, s), 8.37 (1H, s), 8.18 (1H, d), 7.95 (1H, d), 7.62-7.70 (2H, m), 7.49-7.55 (2H, m), 7.33 (1H, t), 4.19 (2H, s), 4. 17 (2H, s); ESI MS *m*/*z* 393 (M+H⁺)

Using essentially the same procedure the following compounds were prepared:
*N-[4-(3-Oxo-3,5-dihydro-pyrazolo[4,3-c]quinolin-2-yl)-phenyl]-succinamic acid*
   ¹H NMR (400 MHz, DMSO-*d*_{*6*}) δ 9.98 (1H, s), 8.67 (1H, s), 8.18 (1H, d), 8.08 (2H, d), 7.58-7.70 (4H, m), 7.51 (1H, t), 2.47-2.57 (4H, m); ESI MS *m*/*z* 377 (M+H⁺).
*4-[4-(3-Oxo-3,5-dihydro-pyrazolo[4,3-c]quinolin-2-yl)-phenylcarbamoyl]-butyric* acid
   ¹H NMR (400 MHz, DMSO-*d*_{*6*}) δ 9.89 (1H, s), 8.67 (1H, s), 8.18 (1H, d), 8.08 (2H, d), 7.60-7.69 (4H, m), 7.51 (1H, t), 2.33 (2H, t), 2.25 (2H, t), 1.75-1.83 (2H, m); ESI MS *m*/*z* 391 (M+H⁺).
*{[4-(3-Oxo-3,5-dihydro-pyrazolo[4,3-c]quinolin-2-yl)-phenylcarbamoyl]-methoxy}-acetic acid*
   ¹H NMR (400 MHz, DMSO-*d*_{*6*}) δ 12.76 (1H, bs), 9.86 (1H, s), 8.68 (1H, d), 8.19 (1H, d), 8.11 (2H, d), 7.61-7.69 (4H, m), 7.52 (1H, t), 4.19 (2H, s), 4.15 (2H, s); ESI MS *m*/*z* 393 (M+H⁺).
*4- [4-(3-Oxo-3,5-dihydro-pyrazolo[4,3-c]quinolin-2-yl)-phenylcarbamoyl]-2-phenyl-butyric acid*
   ¹H NMR (400 MHz, DMSO-*d*_{*6*}) (contain some 3-phenyl regioisomer) δ 9.86 (1H, s), 8.67 (1H, s), 8.18 (1H, d), 8.07 (2H, d), 7.58-7.69 (4H, m), 7.51 (1H, t), 7.20-7.38 (5H, m), 3.55 (1H, t), 2.10-2.30 (3H, m), 1.90-2.02 (1H, m); ESI MS *m*/*z* 467 (M+H⁺).
*N-[3-(3-Oxo-3,5-dihydro-pyrazolo[4,3-c]quinolin-2-yl)-phenyl]-succinamic acid*
   ¹H NMR (400 MHz, DMSO-*d*_{*6*}) δ 10.05 (1H, s), 8.68 (1H, s), 8.31 (1H, s), 8.17 (1H, d), 7,90 (1H, d), 7.62-7.70 (2H, m), 7.48-7.55 (2H, m), 7.30 (1H, t), 2.49-2.57 (4H, m); ESI MS *m*/*z* 377 (M+H⁺).
*2-{[4-(3-Oxo-3,5-dihydro-pyrazolo[4,3-c]quinolin-2-yl)-phenylcarbamoyl]-methyl}-benzoic acid*
   ¹H NMR (400 MHz, DMSO-*d*_{*6*}) δ 10.11 (1H, s), 8,67 (1H, s), 8.18 (1H, d), 8.08 (2H, d), 7.85 (1H, d), 7.59-7.69 (4H, m), 7.46-7.54 (2H, m), 7.35 (2H, t), 4.07 (2H, s); ESI MS *m*/*z* 439 (M+H⁺).

### Example 2

### 4-(3-oxo-3,5-dihydro-pyrazolo[4,3-c]quinolin-2-yl)-benzoic acid

A solution of 4-chloro-quinoline-3-carboxylic acid ethyl ester (11.8 mg, 0.5 mmol) and 4-hydrazino-benzoic acid (7.6 mg, 0.5 mmol) in n-butanol (0.5 mL) was stirred at 115°C over night in a sealed tube. After cooling to 50-70°C, heptane (1.0 mL) was added and the product was allowed to crystallize upon further cooling to room temperature. The solvent was removed and the product was washed with heptane and dried under vacuum to yield 4-(3-oxo-3,5-dihydro-pyrazolo[4,3-*c*]quinolin-2-yl)-benzole acid (13 mg). ¹H NMR (400 MHz, DMSO-*d*_{*6*}) δ 12.9 (1H, s), 8.77 (1H, d), 8.38 (2H, d), 8.25 (1H, d), 8.03 (2H, d), 7.65-7.77 (2H, m), 7.58 (1H, t).

Using essentially the same procedure the following compounds were prepared:
*3-(3-Oxo-3,5-dihydro-pyrazolo[4,3-c]quinolin-2-yl)-benzoic acid*
   ¹H NMR (400 MHz, DMSO-*d*_{*6*}) δ 12.87 (1H, bs), 8.78 (1H, s), 8.72 (1H, d), 8.46 (1H, d), 8.23 (1H, d), 7.63-7.72 (3H, m), 7.51-7.56 (2H, m); ESI MS *m*/*z* 306 (M+H⁺).
*2-Chloro-4-(3-oxo-3,5-dihydro-pyrazolo[4,3-c]quinolin-2-yl)-benzoic acid*

In this reaction the hydrochloride salt of 2-chloro-4-hydrazino-benzoic acid was used and therefor Et₃N (2 eq) was also added to the mixture. ¹H NMR (400 MHz, DMSO-*d*_{*6*}) δ 13.00 (1H, bs), 8.76 (1H, d), 8.44 (1H, s), 8.22-8.28 (2H, m), 7.94 (1H, d), 7.65-7.74 (2H, m), 7.55 (1H, t); ESI MS *m*/*z* 340 (M+H⁺).
*4-(6-Methyl-3-oxo-3,5-dihydro-pyrazolo[4,3-c]quinolin-2-yl)-benzoic acid*
   ¹H NMR (400 MHz, DMSO-*d*_{*6*}) δ 12.1 (1H, s), 8.52 (1H, d), 8.38 (2H, d), 8.12 (1H, d), 8.03 (2H, d), 7.56 (1H, d), 7.48 (1H, t), 2.58 (3H, s).
*4-(8-Methyl-3-oxo-3,5-dihydro-pyrazolo[4,3-c]quinolin-2-yl)-benzoic acid*
   ¹H NMR (400 MHz, DMSO-*d*_{*6*}) δ 12.9 (1H, s), 8.73 (1H, d), 8.39 (2H, d), 8.06 (1H, s), 8.03 (2H, d), 7.64 (1H, d), 7.53 (1H, d), 2.50 (3H, s, in DMSO signal).
*4-(6,8-Dimethyl-3-oxo-3,5-dihydro-pyrazolo[4,3-c]quinolin-2-yl)-benzoic acid*
   ¹H NMR (400 MHz, DMSO-*d*_{*6*}) δ 8.48 (1H, s), 8.38 (2H, d), 8.03 (2H, d), 7.92 (1H, s), 7.40 (1H, s), 2.57 (3H, s), 2.45 (3H, s).
*4-(8-tert-Butyl-3-oxo-3,5-dihydro-pyrazolo[4,3-c]quinolin-2-yl)-benzoic acid*
   ¹H NMR (400 MHz, DMSO-*d*_{*6*}) δ 12.9 (1H, s), 8.75 (1H, d), 8.40 (2H, d), 8.17 (1H, s), 8.03 (2H, d), 7.81 (1H, d), 7.69 (1H, d), 1.40 (9H, s).
*4-(8-Methoxy-6-methyl-3-oxo-3,5-dihydro-pyrazolo[4,3-c]quinolin-2-yl)-benzoic* acid
   ¹H NMR (400 MHz, DMSO-*d*_{*6*}) δ 12.3 (1H, s), 8.45 (1H, d), 8.41 (2H, d), 8.03 (2H, d), 7.49 (1H, d), 7.21 (1H, d), 3.93 (3H, s), 2.57 (3H, s).
*4-(8-Methoxy-3-oxo-3,5-dihydro-pyrazolo[4,3-c]quinolin-2-yl)-benzoic acid*
   ¹H NMR (400 MHz, DMSO-*d*_{*6*}) δ 12.9 (1H, s), 8.71 (1H, d), 8.41 (2H, d), 8.03 (2H, d), 7.70 (1H, d), 7.61 (1H, d), 7.32 (1H, dd), 3.94 (3H, s).
*4-(6,8-Dimethoxy-3-oxo-3,5-dihydro-pyrazolo[4,3-c]quinolin-2-yl)-benzoic acid*
   ¹H NMR (400 MHz, DMSO-*d*_{*6*}) δ 8.40 (2H, d), 8.31 (1H, s), 8.03 (2H, d), 7.18 (1H, d), 6.93 (1H, d), 4.03 (3H, s), 3.95 (3H, s).
*4-(7,9-Dimethoxy-3-oxo-3,5-dihydro-pyrazolo[4,3-c]quinolin-2-yl)-benzoic acid*
   ¹H NMR (400 MHz, DMSO-*d*_{*6*}) δ 12.6 (s, 1H), 8.61 (1H, s), 8.36 (2H, d), 8.01 (2H, d), 6.79 (1H, d), 6.69 (1H, d), 4.00 (3H, s), 3.87 (3H, s).
*4-(3-oxo-8-trifluoromethyl-3,5-dihydro-pyrazolo[4,3-c]quinolin-2-yl)-benzoic acid (60% pure by NMR)*
   ¹H NMR (400 MHz, DMSO-*d*_{*6*}) δ 13.1 (1H, s), 8.85 (1H, d), 8.38 (2H, d), 8.10 (1H, s), 8.03 (2H, d), 7.87 (1H, d), 7.73 (1H, d).
*4-(6-Fluoro-3-oxo-3,5-dihydro-pyrazolo[4,3-c]quinolin-2-yl)-benzoic* acid
   ¹H NMR (400 MHz, DMSO-*d*_{*6*}) δ 13.0 (1H, s), 8.58 (1H, s), 8.37 (2H, d), 8.06 (1H, d), 8.04 (2H, d), 7.54-7.66 (2H, m).
*4-(6-Chloro-3-oxo-3,5-dihydro-pyrazolo[4,3-c]quinolin-2-yl)-benzoic acid*
   ¹H NMR (400 MHz, DMSO-*d*_{*6*}) δ 8.47 (1H, s), 8.36 (2H, d), 8.24 (1H, d), 8.04 (2H, d), 7.88 (1H, d), 7.57 (1H, t).
*4-(8-Chloro-3-oxo-3,5-dihydro-pyrazolo[4,3-c]quinolin-2-yl) -benzoic acid (85% pure by NMR)*
   ¹H NMR (400 MHz, DMSO-*d*_{*6*}) δ 13.0 (1H, s), 8.82 (1H, d), 8.38 (2H, d), 8.20 (1H, s), 8.03 (2H, d), 7.76 (2H, s).
*4- (7, 9-Dichloro-3-oxo-3,5-dihydro-pyrazolo[4,3-c]quinolin-2-yl)-benzoic acid (60% pure by NMR)*
   ¹H NMR (400 MHz, DMSO-*d*_{*6*}) δ 13.0 (s, 1H), 8.81 (1H, s), 8.36 (2H, d), 8.04 (2H, d), 7.78 (1H, d), 7.71 (1H, d).

### Example 3

### [4-(3-Oxo-3,5-dihydro-pyrazolo[4,3-c]quinolin-2-yl)-phenyl]-propionic acid

A suspension of 4-chloro-quinoline-3-carboxylic acid ethyl ester (0.050 g, 0.21 mmol) and 3-(4-hydrazino-phenyl)-propionic acid (0.046 g, 0.25 mmol) in 2-propanol (3 ml) was heated at 84°C in an oil bath for 10 h. The precipitate was collected, washed with 2-propanol and dried to afford the 2-propylester of the product (0.031 g). ¹H NMR (400 MHz, DMSO-d₆) δ 12.8 (1H, bs), 8.66 (1H, d), 8.18 (1H, d), 8.07 (2H, d), 7.60-7.72 (2H, m), 7.52 (1H, t), 7.26 (2H, d), 4.85 (1H, m), 2.82 (2H, t), 2.55 (2H, t), 1.12 (6H, d).

The ester (0.019 g) was hydrolysed by dissolving it in EtOH (0.8 ml) and 1M NaOH (0.4 ml). After 90 minutes the mixture was acidified with 2 M HCl and the precipitate was collected, washed with water and dried to yield [4-(3-oxo-3,5-dihydro-pyrazolo[4,3-*c*]quinolin-2-yl)-phenyl]-propionic acid (0.016 g): ¹H NMR (400 MHz, DMSO-*d*₆) δ 8.66 (1H, s), 8.18 (1H, d), 8.07 (2H, d), 8.61-8.69 (2H, m), 7.51 (1H, t), 7.26 (2H, d), 2.81 (2H, t), 2.52 (2H, t); ESI MS *m*/*z* 334 (M+H⁺).

Using essentially the same procedure the following compounds were prepared:
*[4-(3-Oxo-3,5-dihydro-pyrazolo[4,3-c]quinolin-2-yl)-phenyl]-acetic acid n-butyl ester*
   ¹H NMR (400 MHz, DMSO-d₆) δ 12.8 (1H, bs), 8.68 (1H, d), 8.18 (1H, d), 8.13 (2H, d), 7.61-7-72 (2H, m), 7.53 (1H, t), 7.30 (2H, d), 4.02 (2H, t), 3.63 (2H, s), 1.52 (2H, m), 1.28 (2H, m), 0.83 (3H, t); and
*[4-(3-Oxo-3,5-dihydro-pyrazolo[4,3-c]quinolin-2-yl)-phenyl]-acetic acid*
   ¹H NMR (400 MHz, DMSO-*d*_{*6*}) δ 8.67 (1H, s), 8.19 (1H, d), 8.11 (2H, d), 7.61-7.69 (2H, m), 7.52 (1H, t), 7.29 (2H, d), 3.54 (2H, s); ESI MS *m*/*z* 320 (M+H⁺).

### Example 4

### 3-(4-Hydrazino-phenyl)-propionic acid

A suspension of 3-(4-aminophenyl)-propionic acid (0.50 g, 3.0 mmol) in concentrated hydrochloric acid (3.5 ml) was treated with sodium nitrite (0.21 g, 3.0 mmol) in H₂O (1.7 ml) at 0°C. The reaction was stirred for 45 minutes after which stannous chloride (1.26 g, 6.7 mmol) in concentrated hydrochloride acid (1.5 ml) was added dropwise at 0°C. The reaction was stirred for 1 h at room temperature. The precipitate was collected and dried to afford the hydrochloride salt (0.54 g). The salt (0.100 g) was dissolved in a small amount of water and made basic with 1M NaOH. The solid material was removed by filtration and the filtrate was acidified with acidic acid to yield a precipitate. The solid was collected, washed with water and dried to afford 3-(4-hydrazino-phenyl)-propionic acid (0.050 g): ¹H NMR (400 MHz, DMSO-*d*_{*6*}) δ 6.90 (2H, d), 6.64 (2H, d), 2.63 (2H, t), 2.39 (2H, t).

Using essentially the same procedure the following compound was prepared:
*2-Chloro-4-hydrazino-benzoic acid hydrochloride*

In this reaction the hydrochloride salt was collected and purified by recrystallization from ethanol: ¹H NMR (400 MHz, DMSO-*d*₆) δ 8.97 (1H, bs), 7.76 (1H, d), 7.03 (1H, s), 6.88 (1H, d).

### Example 5

### 4-[3-(2-Hydroxy-phenyl)-5-oxo-4,5-dihydro-pyrazolo-1-yl]-benzonitrile

A suspension of 4-hydroxycoumarine (0.71 g, 4.4 mmol) and 4-cyanophenylhydrazine (0.88 g, 6.6 mmol) in dry toluene was heated at 125°C in an oil bath. Toluene was slowly distilled off during the reaction to separate water. A total of 30 ml toluene was removed. After 3 h the solution was allowed to cool and the precipitate was filtered, washed with toluene and dried. The crude product was dissolved in CH₂Cl₂. The solid material was removed by filtration before the solution was washed with 2M HCl. The organic phase was dried, filtrated and the solvent evaporated to yield 4-[3-(2-hydroxy-phenyl)-5-oxo-4,5-dihydro-pyrazolo-1-yl]-benzonitrile (0.44 g): ¹H NMR (400 MHz, CDCl₃) δ 9.86 (1H, s), 8.01 (2H, d), 7.73 (2H, d), 7.41 (1H, t), 7.23-7.26 (1H, m), 7.08 (1H, d), 6.98 (1H, t), 3.99 (2H, s).

### Example 6

### 4-(4-Methyl-3-oxo-3H-chromeno[4,3-c]pyrazol-2-yl)-benzonitrile

A mixture of 4-[3-(2-hydroxy-phenyl)-5-oxo-4,5-dihydro-pyrazolo-1-yl]-benzonitrile (0.25 g, 0.9 mmol) and triethyl orthoacetate (1.6 ml) was heated at 120°C in an oil bath for 15 minutes. After cooling the precipitate was filtered, washed with diethyl ether and dried to yield 4-(4-methyl-3-oxo-3*H*-chromeno[4,3-*c*]pyrazol-2-yl)-benzonitrile (0.22 g): ¹H NMR (400 MHz, CDCl₃) δ 8.35 (2H, d), 8.18 (1H, d), 7.70 (2H, d), 7.58-7.62 (1H, m), 7.47-7.51 (2H, m), 2.84 (3H, s); ESI MS m/z 302 (M+H⁺).

### Example 7

### 4-(4-Methyl-3-oxo-3H-chromeno[4,3-c]pyrazol-2-yl)-benzoic acid

A mixture of 4-(4-methyl-3-oxo-3*H*-chromeno[4,3-*c*]pyrazol-2-yl)-benzonitrile (0.030 g, 0.10 mmol), acetic acid (0.4 ml), H₂SO₄ (0.4 ml) and water (0.4 ml) was heated at 100°C in an oil bath for 19 h. After cooling water was added and the precipitation collected, washed with water and dried to yield 4-(4-methyl-3-oxo-3*H*-chromeno-[4,3-*c*]pyrazol-2-yl)-benzoic acid (0.029 g) : ¹H NMR (400 MHz, DMSO-*d*₆) δ 8.21 (2H, d), 8.13 (1H, d), 8.01 (2H, d), 7.67-7.75 (2H, m), 7.54-7.58 (1H, m), 2.78 (3H, s); ESI MS *m*/*z* 321 (M+H⁺).

Using essentially the same procedure the following compound was prepared:
*4-(3-Oxo-3H-thiochromeno[4,3-c]pyrazol-2-yl)benzoic acid:*
   ¹H NMR (400 MHz, DMSO-*d*₆) δ 9.29 (1H, s), 8.44 (1H, d), 8.24 (2H, d), 8.03 (2H, d), 7.98 (1H, d), 7.67-7.76 (2H, m); ESI MS *m*/*z* 323 (M+H⁺).

### Example 8

### 4-Oxo-thiochroman-3-carboxylic acid methyl ester

LHMDS (1.12 g, 6.7 mmol) dissolved in anhydrous THF (7 ml) was cooled to -78°C under N₂. Thiochroman-4-one (1.00 g, 6.1 mmol) in anhydrous THF (20 ml) was added dropwise under 20 minutes. After an additional 60 minutes methyl cyanoformate (0.62 g, 7.3 mmol) in anhydrous THF (1.5 ml) was added dropwise under 5 minutes and the suspension was then stirred at -78°C for 80 minutes. The suspension was poured onto 10% NH₄Cl and extracted with ether. The organic phase was washed with water, dried (Na₂SO₄), filtered and the solvent evaporated. The crude product was purified by chromatography on silica gel (heptane-ethyl acetate 10:1) to yield 4-oxo-thiochroman-3-carboxylic acid methyl ester (0.65 g): ¹H NMR (400 MHz, CDCl₃; enol tautomer) δ 12.64 (1H, s), 7.83 (1H, d), 7.25-7.29 (2H, m). 7.16-7.20 (1H, m), 3.84 (3H, s), 3.71 (2H, s).

### Example 9

### 4-(3-Oxo-1,4-dihydro-3H-thiochromeno[4,3-c]pyrazol-2-yl)-benzonitrile

4-Oxo-thiochroman-3-carboxylic acid methyl ester (0.200 g, 0.90 mmol), 4-cyanophenylhydrazine (0.132 g, 0.99 mmol) together with a small amount of pivalic acid was heated at 118°C in an oil bath under N₂. After 1 h the mixture was cooled to room temperature and then triturated with ether. The precipitate was filtered and dried to yield 4-(3-oxo-1,4-dihydro-3*H*-thiochromeno[4,3-*c*]pyrazol-2-yl)-benzonitrile (0.233 g): ¹H NMR (400 MHz, DMSO-*d*₆) δ 8.06 (2H, d), 7.92 (2H, d), 7.80-7.83 (1H, m), 7.30-7.33 (1H, m), 7.18-7.25 (2H, m), 3.91 (2H, s).

### Example 10

### 4- (3-Oxo-3H-thiochromeno[4,3-c]pyrazol-2-yl)-benzonitrile

A solution of 4-(3-oxo-1,4-dihydro-3*H-*thiochromeno[4,3-*c*]pyrazol-2-yl)-benzonitrile (0.100 g, 0.33 mmol) in DMSO (2 ml) was stirred vigorously at room temperature and air was flushed over the solution. After 48 h the precipitation was filtered, washed with toluen and dried. The crude product recrystallized from toluene to yield 4-(3-oxo-3*H*-thiochromeno[4,3-*c*]pyrazol-2-yl)-benzonitrile (0.036 g) : ¹H NMR (400 MHz, CDCl₃) δ 8.65 (1H, s), 8.53 (1H, d), 8.38 (2H, d), 7.72 (2H, d), 7.66 (2H, t), 7.58-7.62 (1H, m); ESI MS m/z 304 (M+H⁺).

### Example 11

### 4-(5-Methyl-3-oxo-3,5-dihydro-pyrazolo[4,3-c]quinolin-2-yl)-benzoic acid

A suspension of 4-(3-oxo-3,5-dihydro-pyrazolo[4,3-c]quinolin-2-yl)-benzoic acid (0.050 g, 0.16 mmol) in DMF (1 ml) was added in small portions to a suspension of NaH (55%) (0.017 g, 0.39 mmol) in DMF (0.5 ml) under N₂. The reaction was stirred at room temperature. After 1 h MeI (0.053 g, 0.38 mmol) was added. After additional 24 h water was added and the solid material (mainly consisting of ester product) was removed by filtration. The filtrate was acidified with 2M HCl. The precipitate was collected, washed with water and dried to yield 4-(5-methyl-3-oxo-3,5-dihydro-pyrazolo[4,3-*c*]quinolin-2-yl)-benzoic acid (0.018 g). This material contained approx. 10% of ester product: ¹H NMR (400 MHz, DMSO-*d*_{*6*}) δ 8.89 (1H, s), 8.34 (2H, d), 8.29 (1H, d), 7.99 (2H, d), 7.85 (1H, d), 7.76 (1H, t), 7.62 (1H, t), 4.00 (3H, s); ESI MS *m*/*z* 320 (M+H⁺).

### Example 12

### 2-[4-(3-Oxo-3,5-dihydro-pyrazolo[4,3-c]quinolin-2-yl)-benzoylamino]-3-phenyl-propionic acid

4-(3-Oxo-3,5-dihydro-pyrazolo[4,3-c]quinolin-2-yl)-benzoic acid (30mg, 0.1mmol) was dissolved in DMF (0.5 ml) and 18mg (0.1mmol) of carbonyldiimidazol (90% pure) was added and the mixture stirred for 4 h. Phenylalanine (33mg, 0.2mmol) was dissolved in of H₂O (0.5 ml) together with triethylamine (0.05 ml) and added to the activated acid. This reaction mixture was heated at 75°C for 2 h. After cooling it was made acidic and diluted 5x with H₂O. The precipitate was filtered and washed with H₂O and dried under vacuum. FlashTube chromatography using EtOAc /MeOH(7:3) yielded a small amount of 2-[4-(3-Oxo-3,5-dihydro-pyrazolo[4,3-c]quinolin-2-yl)-benzoylamino]-3-phenyl-propionic acid. The material contained approx. 10% of starting material. ¹H NMR (400 MHz, DMSO-*d*_{*6*}) δ 8.7 (1H, s), 8.31 (2H, d), 8.21 (1H, d), 7.82 (2H, d), 7.70 (1H, d), 7.62 (1H, t), 7.50 (1H, t), 7.2-7.3 (4H, m), 7.17 (1H, t), 4.50 (1H, m), 3.1-3.2 (2H,m).

Using essentially the same procedure the following compound was prepared:
*2-[4-(3-Oxo-3,5-dihydro-pyrazolo[4,3-c]quinolin-2-yl)-benzoylamino]-2-acetic acid*
   ¹H NMR (400 MHz, DMSO-*d*_{*6*}) δ 8.7 (1H, s), 8.36 (2H, d), 8.22 (1H, d), 7.92 (2H, d), 7.70 (1H, d), 7.63 (1H, t), 7.51 (1H, t), 3.85 (2H,d).

### Example 13

Time resolved fluorescence competition assay protocol: CD28-CD80 interactions.

| Reagent | Supplier |
|---|---|
| Eu-labelled anti-Rabbit antibody (αR-Eu) | Wallac Oy, Turku, Finland, |
| Rabbit anti-mouse IgG, Fc fragment specific (RamIg(Fc)) | Jackson Immunoresearch Laboratories Inc., |
| Human CD28-mouse IgG1 (Fc) (CD28-mFc) | Active Biotech Research AB |
| Human CD80-mouse C215 Fab (C215Fab-hCD80) | Active Biotech Research AB |
| Mouse C215Fab | Active Biotech Research AB |
| Biotin conjugated Goat anti-mouse IgG Kappa light chain (GαMk-biot) | Southern Biotechnology Ass. Inc. |
| Streptavidinallophycocyanin (SA-APC) | Wallac Oy, Turku, Finland |

### Assay buffer

50 mM Tris-HCl, 150 mM NaCl, 0.05% Tween 20, pH 7.8, containing 0.1% BSA (w/v), added prior to use.

### Sample preparation

The substances to be tested for inhibitory effects were serially diluted from stock concentrations of 20 mM (DMSO) in assay buffer to preparation concentrations of 200, 100, 50 and 25 µM. The final concentration in the wells was 100, 50, 25 and 12.5 µM, respectively. The maximum final DMSO concentration tolerated in the assay was 0.5%. Where appropriate, the dilution series was adjusted and extended to measure the IC₅₀. Control

CTLA4-hIg(Fc) was diluted to working concentrations of 20nM and 2nM, resulting in final concentrations of 10nM and 1nM in the well. At these concentrations (10nM and 1nM) approximately 90% and 30% inhibition levels were observed.

### Preparation of reagent mixture

### For 1 plate:

To a tube containing 1.1 ml assay buffer, the following reagents were added:

| Reagent | Volume/ 1.1 ml assay buffer | Preparation conc | Final conc in well |
|---|---|---|---|
| αR-Eu | 4.2 µl from 0.528 mg/ml | 2 µg /ml | 1 µg /ml |
| RαmFc | 2.8 µl from 2.4 mg/ml | 6 µg /ml | 3 µg /ml |
| CD28-mFc | 2.6 µl from 0.4 mg/ml | 0.95 µg/ml (10 nM) | 0.48 µg/ml (5 nM) |
| Gαmκ-biotin | 8.8 µl from 0.5 mg/ml | 4 µg/ml | 2 µg/ml |
| SA-APC | 15.4 µl from 1 mg/ml | 16 µg/ml | 8 µg/ml |

### CD80FabC215 mixture

900 µl of the above reagent mixture was transferred to a new tube. CD80FabC215 was added at a preparation concentration of 20 nM, (i.e. 4.3 µl from 0.4mg/ml stock). The final concentration in the well was 10 nM.

### FabC215 mixture

To the remaining 200 µl reagent mixture C215Fab was added as control for non-specific binding (NSB). 0.5 µl from a 0.36 mg/ml stock gave a preparation concentration of 20nM and a final concentration in the well of 10 nM.

### Pipetting

To a black, half area, 96-well microtiter plate, the C215Fab containing reagent mixture was transferred to column 12, row E-H (4 wells), 10 µl/ well with a single channel electronic pipette. 10µl/well of the CD80FabC215 containing reagent mixture was transferred with a single channel electronic pipette to all other wells on the plate
B0: Assay buffer, 10 µl/well was pipetted to the CD80FabC215 containing wells in column 12, row A-D (4 wells).
NSB: Assay buffer, 10ul/well was pipetted to the C215Fab containing wells in column 12, row E-H (4 wells).
CTLA4-hIg(Fc), 10 µl/well of 20 and 2nM (final conc 10 and 1nM) was pipetted in duplicate into well 11 E-F and 11 G-H.
Sample dilution series: 10 µl/well in duplicate to the remaining wells.

### Incubation

The plate was covered with a plastic lid and incubated in the dark: Initially for 1 h on a shaker platform at room temperature (RTS), stationary at + 4°C overnight, and finally 1 h RTS before reading.

### Measurement

The plate was measured on a Victor 1420 Multilabel Counter using the LANCE protocol (#2) measuring emission at dual wavelengths, from both APC (665nm) and Europium (615nm). First measurement: Excitation 340nm, emission 665nm, delay 50 µs, window time 200 µs. Second measurement: Excitation 340nm, emission 615nm, delay 50 µs, window time 200µs.

### Calculation

The fluorescence signal ratio 1000* 665nm/615nm, from which the percentage inhibition was calculated, was determined. A logit b plot (Logit b = LN (%Bound / (100% - % Bound, plotted against Log conc) was performed from which IC₅₀ was measured.

The assay was performed at least twice in order to have two comparable IC₅₀ values of the compounds.

### Results

The following representative results were obtained:
4-(3-oxo-3,5-dihydro-pyrazolo[4,3-*c*]quinolin-2-yl)-benzoic acid
   IC₅₀ = 0,48 µM
2-Chloro-4-(3-oxo-3,5-dihydro-pyrazolo[4,3-*c*]quinolin-2-yl)-benzoic acid
   IC₅₀ = 0,27 µM
3-(3-Oxo-3,5-dihydro-pyrazolo[4,3-*c*]quinolin-2-yl)-benzoic acid
   IC₅₀ = 0,88 µM
*N*-[4-(3-Oxo-3,5-dihydro-pyrazolo[4,3-*c*]quinolin-2-yl)-phenyl]-succinamic acid
   IC₅₀ = 0, 6 µM
[4-(3-Oxo-3,5-dihydro-pyrazolo[4,3-*c*]quinolin-2-yl)-phenyl]-propionic acid
   IC₅₀ = 1, 6 µM
2-[4-(3-Oxo-3,5-dihydro-pyrazolo[4,3-c]quinolin-2-yl)-benzoylamino]-2-acetic acid
   IC₅₀ = 3,9 µM
4-(3-Oxo-3*H*-thiochromeno[4,3-*c*]pyrazol-2-yl)benzoic acid
   IC₅₀ = 13,5 µM

## Claims

1. A compound having the general formula (I) 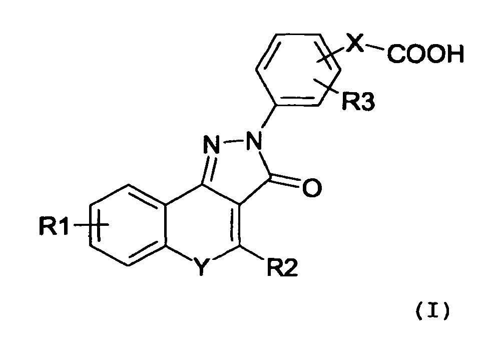 wherein X represents a bond or a group selected from substituted or unsubstituted C₁₋₃-alkyl, NH-C(O)-C₁₋₃₋alkyl, NH-C(O)-CH₂-O-CH₂ or C(O)-NH-(amino acid residue); Y represents NR4, O or S;
R1 represents H, halo, CF₃, lower alkyl or lower alkoxy; R2 and R4 represents independently H or lower alkyl; and R3 represents H, halo, lower alkyl or lower alkoxy,
wherein halo is F, Cl or Br;
wherein lower alkyl represents saturated or unsaturated, straight, branched or cyclic alkyl groups having 1-6 carbon atoms; and
wherein lower alkoxy represents saturated or unsaturated, straight, branched or cyclic alkoxy groups having 1-6 carbon atoms,
with the proviso that R2 is not H, when X is a bond, Y is NH and R3 is H,
or pharmaceutically acceptable salts thereof.

2. A compound according to claim 1, wherein X is a bond.

3. A compound according to claim 1 or 2, wherein Y is NH.

4. A compound according to any one of claims 1-3, selected from the group comprising
{[3-(3-Oxo-3,5-dihydro-pyrazolo[4,3-*c*]quinolin-2-yl)-phenylcarbamoyl]-methoxy}-acetic acid,
*N*-[4-(3-Oxo-3,5-dihydro-pyrazolo[4,3-*c*]quinolin-2-yl)-phenyl]-succinamic acid,
4-[4-(3-Oxo-3,5-dihydro-pyrazolo[4,3-*c*]quinolin-2-yl)-phenylcarbamoyl]-butyric acid,
{[4-(3-Oxo-3,5-dihydro-pyrazolo[4,3-*c*]quinolin-2-yl)-phenylcarbamoyl]-methoxy}-acetic acid,
4-[4-(3-Oxo-3,5-dihydro-pyrazolo[4,3-*c*]quinolin-2-yl)-phenylcarbamoyl]-2-phenyl-butyric acid,
*N*-[3-(3-Oxo-3,5-dihydro-pyrazolo[4,3-*c*]quinolin-2-yl)-phenyl]-succinamic acid,
2-{[4-(3-Oxo-3,5-dihydro-pyrazolo[4,3-*c*]quinolin-2-yl)-phenylcarbamoyl]-methyl}-benzoic acid,
2-Chloro-4-(3-oxo-3,5-dihydro-pyrazolo[4,3-*c*]quinolin-2-yl)-benzoic acid,
4-(6,8-Dimethyl-3-oxo-3,5-dihydro-pyrazolo[4,3-*c*]quinolin-2-yl)-benzoic acid,
4-(8-Methoxy-6-methyl-3-oxo-3,5-dihydro-pyrazolo[4,3-*c*]quinolin-2-yl)-benzoic acid,
4-(6,8-Dimethoxy-3-oxo-3,5-dihydro-pyrazolo[4,3-*c*]quinolin-2-yl)-benzoic acid,
4-(7,9-Dimethoxy-3-oxo-3,5-dihydro-pyrazolo[4,3-*c*]quinolin-2-yl)-benzoic acid,
4-(6-Methyl-3-oxo-8-trifluoromethyl-3,5-dihydro-pyrazolo[4,3-*c*]quinolin-2-yl)-benzoic acid,
4-(7,9-Dichloro-3-oxo-3,5-dihydro-pyrazolo[4,3-*c*]quinolin-2-yl)-benzoic acid,
[4-(3-Oxo-3,5-dihydro-pyrazolo[4,3-*c*]quinolin-2-yl)-phenyl]-propionic acid,
[4-(3-Oxo-3,5-dihydro-pyrazolo[4,3-*c*]quinolin-2-yl)-phenyl]-acetic acid,
4-(4-Methyl-3-oxo-3*H*-chromeno[4,3-*c*]pyrazol-2-yl)-benzoic acid,
4-(3-Oxo-3*H*-thiochromeno[4,3-*c*]pyrazol-2-yl)benzoic acid,
4-(5-Methyl-3-oxo-3,5-dihydro-pyrazolo[4,3-*c*]quinolin-2-yl)-benzoic acid,
2-[4- (3-Oxo-3,5-dihydro-pyrazolo[4,3-c]quinolin-2-yl)-benzoylamino]-3-phenyl-propionic acid, and
2-[4- (3-Oxo-3,5-dihydro-pyrazolo[4,3-c]quinolin-2-yl)-benzoylamino]-2-acetic acid.

5. A compound according to any one of claims 1-4, which is a CD80 antagonist, capable of inhibiting the interaction between CD80 and CD28.

6. A compound according to any one of claims 1-5 for use as a medicament.

7. A compound according to claim 6 for use as a medicament for treatment of medical conditions chosen from the group comprising rheumatoid arthritis, multiple sclerosis, diabetes, asthma, transplantation, systemic lupus erythematosis and psoriasis.

8. Use of a compound as defined in any one of claims 1-5 as a prodrug, preferably in the form of an ester.

9. A pharmaceutical composition comprising a compound according to any one of claims 1-6 as active ingredient in association with a pharmaceutically acceptable adjuvant, diluent or carrier.

10. A pharmaceutical composition according to claim 9 for treatment of medical conditions chosen from the group comprising rheumatoid arthritis, multiple sclerosis, diabetes, asthma, transplantation, systemic lupus erythematosis and psoriasis.

11. Use of a compound having the general formula (I) 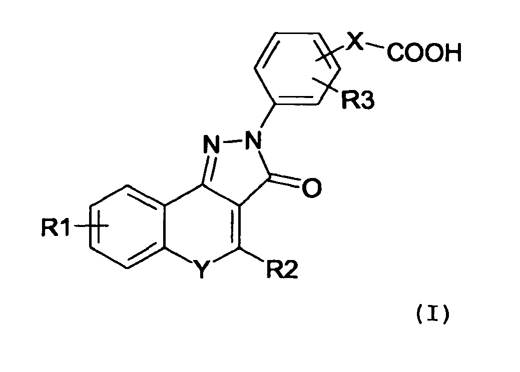 wherein X represents a bond or a group selected from substituted or unsubstituted C₁₋₃-alkyl, NH-C(O)-C₁₋₃₋alkyl, NH-C(O)-CH₂-O-CH₂ or C(O)-NH-(amino acid residue); Y represents NR4, O or S;
R1 represents H, halo, CF₃, lower alkyl or lower alkoxy; R2 and R4 represents independently H or lower alkyl; and R3 represents H, halo, lower alkyl or lower alkoxy,
wherein halo is F, Cl or Br;
wherein lower alkyl represents saturated or unsaturated, straight, branched or cyclic alkyl groups having 1-6 carbon atoms; and
wherein lower alkoxy represents saturated or unsaturated, straight, branched or cyclic alkoxy groups having 1-6 carbon atoms, or pharmaceutically acceptable salts thereof, for the manufacture of a medicament for treatment of medical conditions which benefit from immunomodulation.

12. Use according to claim 11, wherein said medical conditions are chosen from the group comprising rheumatoid arthritis, multiple sclerosis, diabetes, asthma, transplantation, systemic lupus erythematosis and psoriasis.

## Patentansprüche

1. Verbindung mit der allgemeinen Formel (I) 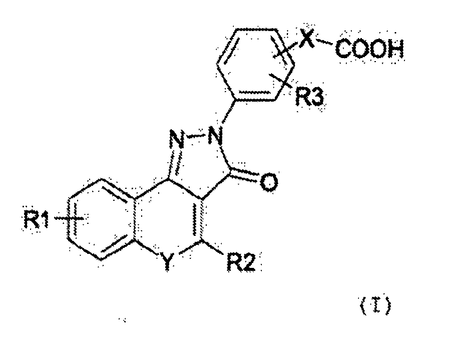 wobei X eine Bindung oder eine Gruppe ausgewählt aus substituiertem oder unsubstituiertem C₁₋₃-Alkyl, NH-C(O)-C₁₋₃-Alkyl, NH-C(O)-CH₂-O-CH₂ oder C(O)-NH-(Aminosäurerest) bedeutet;
Y NR4, O oder S bedeutet;
R1 H, Halogen, CF₃, niederes Alkyl oder niederes Alkoxy bedeutet;
R2 und R4 unabhängig voneinander H oder niederes Alkyl bedeutet; und
R3 H, Halogen, niederes Alkyl oder niederes Alkoxy bedeutet, wobei Halogen F, Cl oder Br ist;
wobei niederes Alkyl gesättigte oder ungesättigte, geradkettige, verzweigte oder cyclische Alkylgruppen mit 1-6 Kohlenstoffatomen bedeutet; und
wobei niederes Alkoxy gesättigte oder ungesättigte, geradkettige, verzweigte oder cyclische Alkoxygruppen mit 1-6 Kohlenstoffatomen bedeutet
mit der Maßgabe, dass R2 nicht H ist, wenn X eine Bindung ist, Y NH ist und R3 H ist,
oder pharmazeutisch annehmbare Salze davon.

2. Verbindung nach Anspruch 1, wobei X eine Bindung ist.

3. Verbindung nach Anspruch 1 oder 2, wobei Y NH ist.

4. Verbindung nach einem der Ansprüche 1-3, ausgewählt aus der Gruppe umfassend
{[3-(3-Oxo-3,5-dihydro-pyrazolo[4,3-c]chinolin-2-yl)-phenylcarbamoyl]-methoxy}-essigsäure,
N-[4-(3-Oxo-3,5-dihydro-pyrazolo[4,3-c]chinolin-2-yl)-phenyl]-succinamidsäure,
4-[4-(3-Oxo-3,5-dihydro-pyrazolo[4,3-c]chinolin-2-yl)-phenylcarbamoyl]-buttersäure,
{[4-(3-Oxo-3,5-dihydro-pyrazolo[4,3-c]chinolin-2-yl)-phenylcarbamoyl]-methoxy}-essigsäure,
4-[4-(3-Oxo-3,5-dihydro-pyrazolo[4,3-c]chinolin-2-yl)-phenylcarbamoyl]-2-phenyl-buttersäure,
N-[3-(3-Oxo-3,5-dihydro-pyrazolo[4,3-c]chinolin-2-yl)-phenyl]-succinamidsäure,
2-{[4-(3-Oxo-3,5-dihydro-pyrazolo[4,3-c]chinolin-2-yl)-phenylcarbamoyl]-methyl}-benzoesäure,
2-Chlor-4-(3-oxo-3,5-dihydro-pyrazolo[4,3-c]chinolin-2-yl)-benzoesäure,
4-(6,8-Dimethyl-3-oxo-3,5-dihydro-pyrazolo[4,3-c]chinolin-2-yl)-benzoesäure,
4-(8-Methoxy-6-methyl-3-oxo-3,5-dihydro-pyrazolo[4,3-c]chinolin-2-yl)-benzoesäure,
4-(6,8-Dimethoxy-3-oxo-3,5-dihydro-pyrazolo[4,3-c]chinolin-2-yl)-benzoesäure,
4-(7,9-Dimethoxy-3-oxo-3,5-dihydro-pyrazolo[4,3-c]chinolin-2-yl)-benzoesäure,
4-(6-Methyl-3-oxo-8-trifluormethyl-3,5-dihydropyrazolo[4,3-c]chinolin-2-yl)-benzoesäure,
4-(7,9-Dichlor-3-oxo-3,5-dihydro-pyrazolo[4,3-c]chinolin-2-yl)-benzoesäure,
[4-(3-Oxo-3,5-dihydro-pyrazolo[4,3-c]chinolin-2-yl)-phenyl]-propionsäure,
[4-(3-Oxo-3,5-dihydro-pyrazolo[4,3-c]chinolin-2-yl)-phenyl]-essigsäure,
4-(4-Methyl-3-oxo-3H-chromeno[4,3-c]pyrazol-2-yl)-benzoesäure,
4-(3-Oxo-3H-thiochromeno[4,3-c]pyrazol-2-yl)benzoesäure,
4-(5-Methyl-3-oxo-3,5-dihydro-pyrazolo[4,3-c]chinolin-2-yl)-benzoesäure,
2-[4-(3-Oxo-3,5-dihydro-pyrazolo[4,3-c]chinolin-2-yl)benzoylamino]-3-phenyl-propionsäure, und
2-[4-(3-Oxo-3,5-dihydro-pyrazolo[4,3-c]chinolin-2-yl)benzoylamino]-2-essigsäure

5. Verbindung nach einem der Ansprüche 1-4, welche ein CD80-Antagonist ist, der imstande ist, die Wechselwirkung zwischen CD80 und CD28 zu hemmen.

6. Verbindung nach einem der Ansprüche 1-5 zur Verwendung als ein Medikament.

7. Verbindung nach Anspruch 6 zur Verwendung als ein Medikament für die Behandlung von medizinischen Zuständen, ausgewählt aus der Gruppe umfassend rheumatoide Arthritis, Multiple Sklerose, Diabetes, Asthma, Transplantation, systemischen Lupus erythematodes und Psoriasis.

8. Verwendung einer Verbindung wie in einem der Ansprüche 1-5 definiert als Prodrug, vorzugsweise in Form eines Esters

9. Pharmazeutische Zusammensetzung, umfassend eine Verbindung nach einem der Ansprüche 1-6 als Wirkstoff in Verbindung mit einem pharmazeutisch annehmbaren Adjuvans, Verdünnungsmittel oder Träger.

10. Pharmazeutische Zusammensetzung nach Anspruch 9 für die Behandlung von medizinischen Zuständen, ausgewählt aus der Gruppe umfassend rheumatoide Arthritis, Multiple Sklerose, Diabetes, Asthma, Transplantation, systemischen Lupus erythematodes und Psoriasis.

11. Verwendung einer Verbindung mit der allgemeinen Formel (I) 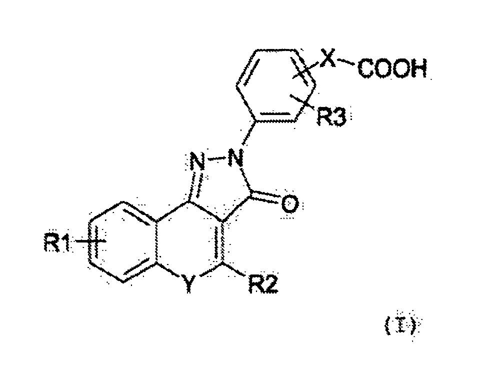 wobei X eine Bindung oder eine Gruppe ausgewählt aus substituiertem oder unsubstituiertem C₁₋₃-Alkyl, NH-C(O)-C₁₋₃-Alkyl, NH-C(O)-CH₂-O-CH₂ oder C(O)-NH-(Aminosäurerest) bedeutet;
Y NR4, O oder S bedeutet;
R1 H, Halogen, CF₃, niederes Alkyl oder niederes Alkoxy bedeutet;
R2 und R4 unabhängig voneinander H oder niederes Alkyl bedeutet; und
R3 H, Halogen, niederes Alkyl oder niederes Alkoxy bedeutet, wobei Halogen F, Cl oder Br ist;
wobei niederes Alkyl gesättigte oder ungesättigte, geradkettige, verzweigte oder cyclische Alkylgruppen mit 1-6 Kohlenstoffatomen bedeutet; und
wobei niederes Alkoxy gesättigte oder ungesättigte, geradkettige, verzweigte oder cyclische Alkoxygruppen mit 1-6 Kohlenstoffatomen bedeutet,
oder pharmazeutisch annehmbarer Salze davon für die Herstellung eines Medikaments für die Behandlung von medizinischen Zuständen, welche aus einer Immunmodulation Nutzen ziehen.

12. Verwendung nach Anspruch 11, wobei die medizinischen Zustände ausgewählt sind aus der Gruppe umfassend rheumatoide Arthritis, Multiple Sklerose, Diabetes, Asthma, Transplantation, systemischen Lupus erythematodes und Psoriasis.

## Revendications

1. Composé ayant la formule générale (I) 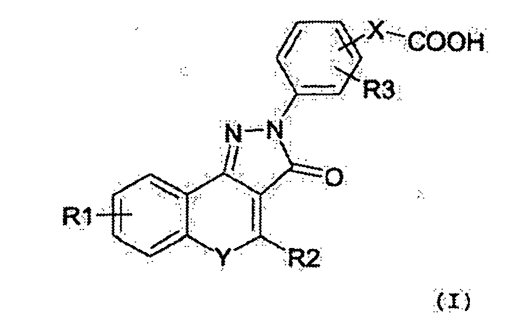 dans laquelle
X représente une liaison ou un groupe choisi parmi alkyle en C₁-C₃ substitué ou non substitué, NH-C(O)-(alkyle en C₁-C₃), NH-C(O)-CH₂-O-CH₂ ou C(O)-NH-(résidu d'aminoacide);
Y représente NR4, O ou S;
R1 représente H, halogéno, CF₃, alkyle inférieur ou alcoxy inférieur;
R2 et R4 représentent indépendamment H ou alkyle inférieur; et
R3 représente H, halogéno, alkyle inférieur ou alcoxy inférieur;
où halogéno est F, Cl ou Br;
où alkyle inférieur représente des groupes alkyle saturés ou insaturés, linéaires, ramifiés ou cycliques ayant 1 - 6 atomes de carbone; et
ou alcoxy inférieur représente des groupes alcoxy saturés ou insaturés, linéaires, ramifiés ou cycliques ayant 1 - 6 atomes de carbone,
à condition que R2 ne soit pas H lorsque X est une liaison, Y est NH et R3 est H,
ou ses sels pharmaceutiquement acceptables.

2. Composé selon la revendication 1, dans lequel X est une liaison.

3. Composé selon la revendication 1 ou 2, dans lequel Y est NH.

4. Composé selon l'une quelconque des revendications 1-3, choisi dans le groupe comprenant
l'acide {[3-(3-oxo-3,5-dihydropyrazolo[4,3-c]quinoléin-2-yl)phénylcarbamoyl]-méthoxy}acétique,
l'acide N-[4-(3-oxo-3,5-dihydropyrazolo[4,3-c]quinoléin-2-yl)phényl]succinamique,
l'acide 4-[4-(3-oxo-3,5-dihydropyrazolo[4,3-c]quinoléin-2-yl)phénylcarbamoyl]-butyrique,
l'acide {[4-(3-oxo-3,5-dihydropyrazolo[4,3-c]quinoléin-2-yl)phénylcarbamoyl]-méthoxy}acétique,
l'acide 4-[4-(3-oxo-3,5-dihydropyrazolo[4,3-c]quinoléin-2-yl)phénylcarbamoyl]-2-phénylbutyrique,
l'acide N-[3-(3-oxo-3,5-dihydropyrazolo[4,3-c]quinoléin-2-yl)phényl]succinamique,
l'acide 2-{[4-(3-oxo-3,5-dihydropyrazolo[4,3-c]quinoléin-2-yl)phénylcarbamoyl]-méthyl}benzoïque,
l'acide 2-chloro-4-(3-oxo-3,5-dihydropyrazolo[4,3-c]quinoléin-2-yl)benzoïque,
l'acide 4-(6,8-diméthyl-3-oxo-3,5-dihydropyrazolo[4,3-c]quinoléin-2-yl)benzoïque,
l'acide 4-(8-méthoxy-6-méthyl-3-oxo-3,5-dihydropyrazolo[4,c]quinoléin-2-yl)-benzoïque,
l'acide 4-(6,8-diméthoxy-3-oxo-3,5-dihydropyrazolo[4,3-c]quinoléin-2-yl)benzoïque,
l'acide 4-(7,9-diméthoxy-3-oxo-3,5-dihydropyrazolo[4,3-c]quinoléin-2-yl)benzoïque,
l'acide 4-(6-méthyl-3-oxo-8-trifluorométhyl-3,5-dihydropyrazolo[4,3-c]quinoléin-2-yl)benzoïque,
l'acide 4-(7,9-dichloro-3-oxo-3,5-dihydropyrazolo[4,3c]quinoléin-2-yl)benzoïque,
l'acide [4-(3-oxo-3,5-dihydropyrazolo[4,3-c]quinoléin-2-yl)phényl]propionique,
l'acide [4-(3-oxo-3,5-dihydropyrazolo[4,3-c]quinoléin-2-yl)phényl]acétique,
l'acide 4-(4-méthyl-3-oxo-3H-chroméno[4,3-c]pyrazol-2-yl)benzoïque,
l'acide 4-(3-oxo-3H-thiochroméno[4,3-c]pyrazol-2-yl) benzoïque,
l'acide 4-(5-méthyl-3-oxo-3,5-dihydropyrazolo[4,3-c]quinoléin-2-yl)benzoïque,
l'acide 2-[4-(3-oxo-3,5-dihydropyrazolo[4,3-c]quinoléin-2-yl)benzoylamino]-3-phénylpropionique, et
l'acide 2-[4-(3-oxo-3,5-dihydropyrazolo[4,3-c]quinoléin-2-yl)benzoylamino]-2-acétique.

5. Composé selon l'une quelconque des revendication 1-4, qui est un antagoniste du CD80, capable d'inhiber l'interaction entre le CD80 et le CD28.

6. Composé selon l'une quelconque des revendications 1-5 à utiliser comme médicament.

7. Composé selon la revendication 6, à utiliser comme médicament destiné au traitement de situations médicales choisies dans le groupe comprenant la polyarthrite rhumatoïde, la sclérose en plaques, le diabète, l'asthme, la transplantation, le lupus érythémateux aigu disséminé et le psoriasis.

8. Utilisation d'un composé selon l'une quelconque des revendications 1-5 comme promédicament, de préférence sous forme d'un ester.

9. Composition pharmaceutique comprenant un composé selon l'une quelconque des revendications 1-6 comme ingrédient actif en association avec un adjuvant, diluant ou support pharmaceutiquement acceptable.

10. Composition pharmaceutique selon la revendication 9, destinée au traitement de situations médicales choisies dans le groupe comprenant la polyarthrite rhumatoïde, la sclérose en plaques, le diabète, l'asthme, la transplantation, le lupus érythémateux aigu disséminé et le psoriasis.

11. Utilisation d'un composé ayant la formule générale (I) 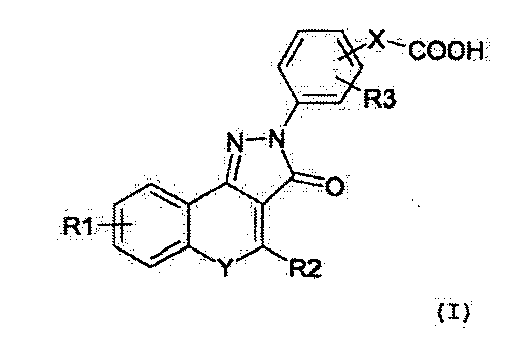 dans laquelle
X représente une liaison ou un groupe choisi parmi alkyle en C₁-C₃ substitué ou non substitué, NH-C(O)-(alkyle en C₁-C₃), NH-C(O)-CH₂-O-CH₂ ou C(O)-NH-(résidu d'aminoacide);
Y représente NR4, O ou S;
R1 représente H, halogéno, CF₃, alkyle inférieur ou alcoxy inférieur;
R2 et R4 représentent indépendamment H ou alkyle inférieur; et
R3 représente H, halogéno, alkyle inférieur ou alcoxy inférieur;
où halogéno est F, Cl ou Br;
où alkyle inférieur représente des groupes alkyle saturés ou insaturés, linéaires, ramifiés ou cycliques ayant 1 - 6 atomes de carbone; et
ou alcoxy inférieur représente des groupes alcoxy saturés ou insaturés, linéaires, ramifiés ou cycliques ayant 1 - 6 atomes de carbone,
ou de ses sels pharmaceutiquement acceptables, pour la fabrication d'un médicament destiné au traitement de situations médicales qui tirent profit d'une immunomodulation.

12. Utilisation selon la revendication 11, dans laquelle lesdites situations médicales sont choisies dans le groupe comprenant la polyarthrite rhumatoïde, la sclérose en plaques, le diabète, l'asthme, la transplantaiton, le lupus érythémateux aigu disséminé et le psoriasis.
